# EUROPÄISCHE PATENTSCHRIFT

(11) **EP 1 542 803 B1**
(45) Veröffentlichungstag und Bekanntmachung des Hinweises auf die Patenterteilung: **07.11.2007**
(21) Anmeldenummer: 03775159.1
(22) Anmeldetag: 18.09.2003
(51) Int. Cl.: B01L 3/00, G01N 33/53

(54) **MEHRFACH-KAPILLARSENSOR-ANALYSESYSTEM**
MULTIPLE CAPILLARY SENSOR ANALYSIS SYSTEM
SYSTEME D'ANALYSE A CAPTEURS CAPILLAIRES MULTIPLES

(30) Priorität: 26.09.2002 DE 10244775
(43) Veröffentlichungstag der Anmeldung: 22.06.2005
(73) Patentinhaber: Roche Diagnostics GmbH, 68305 Mannheim (DE); F. HOFFMANN-LA ROCHE AG, 4070 Basel (CH)
(72) Erfinder: MARQUANT, Michael, 68309 Mannheim (DE)
(74) Vertreter: Pfeifer, Hans-Peter
(86) Internationale Anmeldenummer: PCT/EP2003/010378
(87) Internationale Veröffentlichungsnummer: WO 2004/030822

(56) Entgegenhaltungen:
- EP-A- 0 826 963
- EP-A- 1 114 995
- US-A- 5 395 504
- US-A- 5 609 823
- US-A- 6 027 689

## Beschreibung

Die Erfindung betrifft ein Kapillarsensor-Analysesystem zur Analyse einer Probenflüssigkeit auf einen darin enthaltenen Analyten sowie einen als Bestandteil eines solchen Systems geeigneten Mehrfach-Kapillarsensor-Streifen.

Zur qualitativen und quantitativen Analyse von Bestandteilen einer flüssigen Probe, insbesondere einer Körperflüssigkeit von Menschen oder Tieren werden in großem Umfang Analysesysteme verwendet, die aus zur einmaligen Verwendung vorgesehenen Analyseelementen und speziell auf einen bestimmten Typ der Analyseelemente abgestimmten Auswertegeräten bestehen. Zur Durchführung einer Analyse wird das Analyseelement, das in der Fachwelt auch als "Biosensor" bezeichnet wird, mit der Probe in Kontakt gebracht. Die Reaktion der Probe mit mindestens einem in dem Biosensor enthaltenen Reagenz führt zu einer für die Analyse charakteristischen Änderung einer meßbaren physikalischen Eigenschaft (Meßgröße) des Biosensors. Das Auswertegerät enthält eine Meß- und Auswerteelektronik zur Messung der Meßgröße und zur Ermittlung der gewünschten Analyseinformation (in der Regel der Konzentration des Analyten) auf Basis des gemessenen Meßwertes.

In der Anfangszeit der Entwicklung waren vor allem Analyseelemente in Form von Teststreifen gebräuchlich, die aus einem Kunststoffträger mit mindestens einem die Reagenzien enthaltenen Testfeld bestehen. Um beispielsweise einen durch einen Stich in einen Finger gewonnenen Blutstropfen zu analysieren, wird das an dem Finger haftende Blut unmittelbar auf das Testfeld aufgebracht. Danach wird der Blutüberschuß abgewischt oder abgewaschen. Die Reaktion des Blutes mit den in dem Testfeld enthaltenden Reagenzien führt zu einer Änderung von dessen Farbe, die mittels eines in dem Auswertegerät enthaltenen Photometers ausgewertet werden kann.

Diese Verfahrensweise hat jedoch einige Nachteile. Insbesondere ist die Variation der in das Testfeld eindringenden und für die Analyse zur Verfügung stehenden Probenmenge relativ hoch. Außerdem ist die Handhabung für manche Benutzer schwierig. Dies gilt insbesondere, soweit das Analysesystem zur Benutzung durch den Patienten selbst ("home monitoring") bestimmt ist, weil diese Benutzer häufig durch Erkrankung und/oder erhöhtes Alter in ihrer Bewegungspräzision eingeschränkt sind.

Um diese Probleme zu überwinden, werden - ebenfalls schon seit vielen Jahren - Kapillarsensoren vorgeschlagen, die einen von mindestens zwei Wandteilen umschlossenen Kapillarkanal mit einer Einlaßöffnung für die Probenflüssigkeit und einer Entlüftungsöffnung aufweisen und in deren Kapillarkanal die für die Analyse erforderlichen Reagenzien enthalten sind. Bei der Benutzung dieser Kapillarsensoren wird ein Tropfen der Probenflüssigkeit (in der Regel Blut) in Kontakt zu der Einlaßöffnung gebracht und durch Kapillarwirkung in den Kapillarkanal gesaugt, in dem die für die Analyse charakteristische Reaktion stattfindet. Dadurch wird eine leichtere Handhabung und eine präzise Dosierung der an der Reaktion beteiligten Probenflüssigkeit erreicht.

Colorimetrische Kapillarsensoren sind beispielsweise in folgenden Publikationen beschrieben:
1) US-Patent 4,088,448
2) GB 2 090 659 A
3) EP 0 057 110

In der Publikation
4) WO 86/00138
wird ein Kapillarsensor beschrieben, bei dem die Analyse auf elektrochemischen Prinzipien basiert. Dabei enthält der Kapillarkanal mindestens zwei Elektroden (Arbeitselektrode und Gegenelektrode) und die Reagenzien sind so ausgewählt, daß als Folge der Analysereaktion eine mittels der Elektroden elektrisch meßbare Veränderung (eines Stromes oder einer Spannung) stattfindet. Um die erforderliche Verbindung mit dem Auswertegerät herzustellen, weisen diese Kapillarsensoren Sensorkontakte auf, die einerseits mit den Elektroden verbunden sind und andererseits beim Einstecken des Kapillarsensors in das Auswertegerät entsprechende Gerätekontakte kontaktieren und dadurch eine elektrische Verbindung zu der Meß- und Auswerteelektronik des Gerätes herstellen. Neuere Ausführungsformen solcher elektrochemischer Kapillarsensoren sind beispielsweise in folgenden Publikationen beschrieben:
5) US-Patent 5,437,999
6) WO 99/32881
7) US-Patent 6,103,033
8) Patent Abstract of Japan 2000-258382
9) US-Patent 6,071,391

Auf dieser Grundlage liegt der Erfindung das technische Problem zugrunde, die Analyse von Körperflüssigkeiten, insbesondere von Blut, mittels Kapillarsensoren, insbesondere hinsichtlich einer einfachen Handhabung, weiter zu verbessern.

Die Aufgabe wird durch einm Kapillarsensor-Analysesystem gemäß Anspruch 1 gelöst.

Bevorzugt arbeitet das erfindungsgemäße System mit elektrochemischen Kapillarsensoren. Eine besonders einfache Handhabung wird erreicht, wenn das Auswertegerät gemäß einer bevorzugten Ausführungsform eine Schneideeinrichtung aufweist, durch die jeweils nach Durchführung einer Messung der bei der Messung benutzte Kapillarsensor von dem Mehrfach-Kapillarsensor-Streifen abgeschnitten wird.

Für konventionelle colorimetrische Analyseelemente ohne Kapillarkanal sind bereits seit langem Konstruktionen bekannt, bei denen eine Mehrzahl von Analyseelementen zu einem Streifen zusammengefaßt sind. Beispielsweise ist in der Publikation
10) EP 0 299 517 A2
   ein Analysesystem beschrieben, bei dem ein streifenförmiger Analysefilm verwendet wird, der eine Vielzahl von mehrschichtigen Analyseelementen enthält. Der streifenförmige Analysefilm wird in dem Gerät auf einer ersten Spule bereitgehalten und nach Benutzung auf eine zweite Spule aufgewickelt. Eine ähnliche Konstruktion ist in der
11) DE 198 19 407 A1
   beschrieben. Beispiele von Mehrfach-Streifen, die aus einer Reihe von miteinander verbundenen colorimetrischen Analyseelementen bestehen, sind aus den Publikationen
12) US-Patent 5,757,666
13) DE 197 14 674 A1
14) US-Patent 6,027,689 und
15) EP 0 826 963 A2
   bekannt. Ein elektrochemischer Mehrfach-Sensorstreifen, dessen einzelne Sensoren jeweils mittels eines in das zugehörige Auswertegerät integrierten Messers abgeschnitten werden können, ist aus dem US-Patent 5,395,504 bekannt. Eine ähnliche Konstruktion wird auch in der EP 1 114 995 beschrieben, wobei dort die Sensoren über Solltrennstellen miteinander verbunden sein können, die die Trennung durch Auseinanderziehen ermöglichen.

In diesem Dokument wird auch eine besondere Form von Kapillarsensoren beschrieben.

Diese Konstruktionen eignen sich jedoch nicht für Kapillarsensoren, bei denen spezifische Probleme bestehen, die beispielsweise aus der Integration des Kapillarkanals, den damit verbundenen Besonderheiten des Herstellungsprozesses und den Erfordernissen der Anwendung (z.B. Zugänglichkeit der Einlaßöffnung) resultieren. Zusätzliche Besonderheiten ergeben sich bei elektrochemischen Kapillarsensoren, beispielsweise durch die Erfordernisse der elektrischen Kontaktierung.

Die Erfindung wird nachfolgend anhand von in den Figuren schematisch dargestellten Ausführungsbeispielen näher erläutert. Die darin dargestellten Besonderheiten können einzeln oder in Kombination verwendet werden, um bevorzugte Ausgestaltungen der Erfindung zu schaffen. Es zeigen:
- Fig. 1: ein erfindungsgemäßes Kapillarsensor-Analyse-system in Seitenansicht, teilweise als Blockschaltbild,
- Fig. 2: einen Mehrfach-Kapillarsensor-Streifen während des Zuführens einer Blutprobe,
- Fig. 3: eine perspektivische Darstellung eines Mehrfach-Kapillarsensor-Streifens,
- Fig. 4: eine teilweise aufgeschnittene perspektivische Darstellung eines Mehrfach-Kapillarsensor-Streifens,
- Fig. 5: einen Querschnitt durch einen Mehrfach-Kapillarsensor-Streifen,
- Fig. 6: eine Darstellung der elektrischen Verbindung zwischen einem Mehrfach-Kapillarsensor-Streifen und der Meß- und Auswerteelektronik,
- Fig. 7: eine perspektivische Darstellung einer alternativen Ausführungsform eines Mehrfach-Kapillarsensor-Streifens mit Schleifkontakten,
- Fig. 8: eine Prinzipdarstellung einer alternativen Ausführungsform eines erfindungsgemäßen Kapillarsensor-Analysesystems,
- Fig. 9: eine Darstellung des Layouts verschiedener Schichten eines Mehrfach-Kapillarsensor-Streifens zur Verwendung in einem System nach Figur 8,
- Fig. 10: eine Detailansicht einer Elektrodenanordnung aus Figur 9.

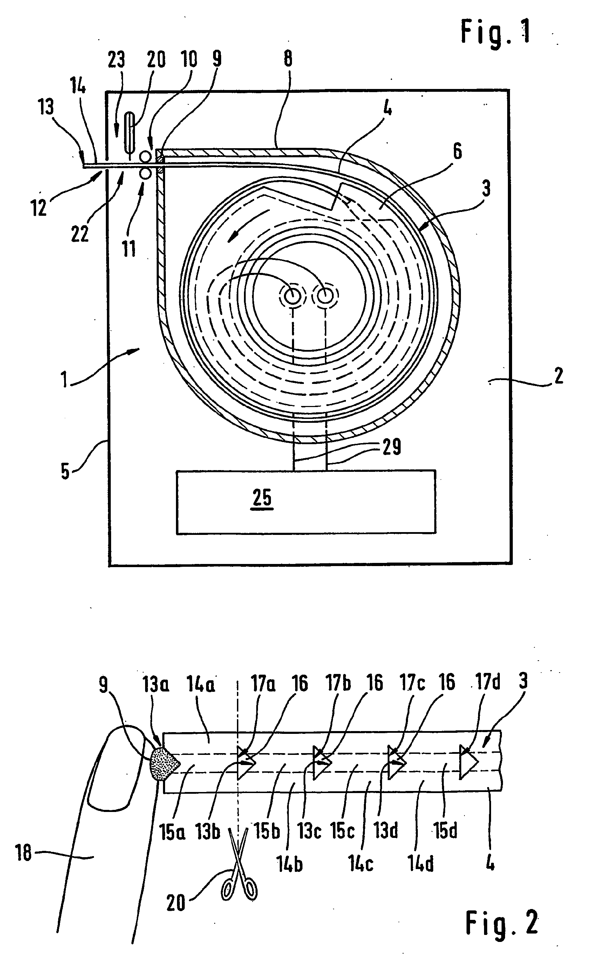

Das in Figur 1 dargestellte Kapillarsensor-Analysesystem 1 besteht aus einem Auswertegerät 2 und Mehrfach-Kapillarsensor-Streifen 3, die auswechselbar in das Auswertegerät 1 eingesetzt werden können. Im dargestellten Fall ist der Mehrfach-Kapillarsensor-Streifen 3 als flexibles Sensorband 4 ausgebildet, das in dem Gehäuse 5 des Auswertegerätes 2 mittels einer hohlen drehbaren Trommel 6 in gekrümmtem Zustand aufgewickelt ist.

Das Sensorband 4 und die Trommel 6 befinden sich innerhalb einer abgedichteten Kassette 8, die auswechselbar in das Auswertegerät 2 eingesetzt werden kann. Das vordere Ende des Sensorbandes 4 ragt durch eine mit flexiblen Dichtungen 9 versehene Austrittsöffnung 10 aus der Kassette 8 heraus, wobei es zwischen zwei Transportrollen 11 eingeklemmt ist und aus einer Öffnung 12 des Gehäuses 2 derartig hervorsteht, daß eine an der Vorderkante des Mehrfach-Kapillarsensor-Streifens vorhandene Einlaßöffnung 13 zugänglich ist, so daß sie mit einer Probenflüssigkeit in Kontakt gebracht werden kann.

Fig. 2 zeigt eine Prinzipskizze dieses Vorgangs. Der in Aufsicht dargestellt Mehrfach-Kapillarsensor-Streifen 3 besteht aus mehreren hintereinander angeordneten Einzel-Kapillarsensoren 14a-14d, die von aufeinanderfolgenden Abschnitten des Sensorbandes 4 gebildet werden. Die Kapillarsensoren 14 enthalten jeweils einen Kapillarkanal 15a-15d mit jeweils einer Einlaßöffnung 13a-13d für die Probenflüssigkeit und einer Entlüftungsöffnung 17a-17d.

Bei der dargestellten bevorzugten Ausführungsform verlaufen die Kapillarkanäle 15 der Kapillarsensoren in Längsrichtung des Mehrfach-Kapillarsensor-Streifens und schließen derartig fluchtend aneinander an, daß ihre Einlaß- und Auslaßöffnungen einander gegenüberliegen. Die erforderliche Trennung der Kapillarkanäle 15 wird durch Strömungshindernisse erreicht, die hier durch Ausnehmungen 16 gebildet werden, die das gesamte Sensorband 4 durchdringen, sich mindestens über die gesamte Breite der Kapillarkanäle 15 erstrecken und zugleich die Einlaßöffnungen 13 und die Entlüftungsöffnungen 17 bilden.

Die dargestellte Ausführungsform mit fluchtend aneinander anschließenden Kapillarkanälen und (infolgedessen) einander gegenüberliegenden Einlaß- und Entlüftungsöffnungen ist produktionstechnisch besonders vorteilhaft. Ein solcher Mehrfach-Kapillarsensor-Streifen kann, wie weiter unten noch näher erläutert wird, auf einfache Weise in einem kontinuierlichen Verfahren hergestellt werden, wobei alle Produktionsschritte an einem in Längsrichtung der Streifen verlaufenden Band durchgeführt werden können, ohne daß Bewegungen quer zu der Produktionsrichtung erforderlich sind. Strömungshindernisse in Form von Ausnehmungen, die mindestens eines der Wandteile durchdringen und sich über die gesamte Breite des Kapillarkanals erstrecken, sind sowohl hinsichtlich der Funktion als auch produktionstechnisch besonders vorteilhaft, jedoch kann ein Strömungshindernis auch auf andere Weise, beispielsweise durch Hydrophobierung des entsprechenden Abschnitts des Kapillarkanals, realisiert werden.

Um eine Analyse durchzuführen, wird ein Tropfen Probenflüssigkeit, beispielsweise ein durch Einstich in einen Finger 18 gewonnener Blutstropfen 19, mit der Einlaßöffnung des vordersten Kapillarsensors des Mehfach-Kapillarsensor-Streifens 3 derartig in Kontakt gebracht, daß die Probenflüssigkeit in den Kapillarkanal des vordersten Sensors 14a des Streifens 3 gesaugt wird. Wenn dessen Kapillarkanal 15a gefüllt ist, findet die Reaktion mit darin enthaltenen Reagenzien und anschließend die Messung einer für die Analyse charakteristischen Meßgröße statt. Hinsichtlich der verwendeten biochemischen Analyseverfahren unterscheidet sich die Erfindung nicht von bekannten Kapillarsensoren, so daß insoweit keine nähere Erläuterung notwendig ist.

Nach der Durchführung der Messung wird der benutzte vorderste Kapillarsensor 14a mittels einer Schneideeinrichtung 20 von dem Mehrfach-Kapillarsensor-Streifen 3 abgeschnitten und entsorgt. Anschließend wird der Mehrfach-Kapillarsensor-Streifen 3 mittels der Transportrollen 11 weitertransportiert, so daß der nächste folgende Kapillarsensor 14b in die Meßposition 22 gebracht wird. Bei der dargestellten Ausführungsform bilden also die Kassette 8 und die Transportrollen 11 eine insgesamt mit 23 bezeichnete Kapillarsensor-Halterung, um jeweils einen Kapillarsensor 14 in der Meßposition 22 derartig zu positionieren, daß seine Einlaßöffnung 13 zugänglich ist, um eine Probe so mit ihr in Kontakt zu bringen, daß die Probenflüssigkeit aufgrund von Kapillarkräften in den Kapillarkanal 15 eindringt und diesen füllt. Der Mehrfach-Kapillarsensor-Streifen 3 wird in der Kapillarsensor-Halterung 23 derartig geführt und gehalten, daß sich jeweils der vorderste Kapillarsensor 14a des Streifens 3 in der Meßposition 22 befindet, in der seine Einlaßöffnung 13a zur Kontaktierung mit Probenflüssigkeit zugänglich ist. Er ist in dem Auswertegerät 2 so bewegbar, daß nacheinander aufeinanderfolgende Kapillarsensoren 14 in die Meßposition 22 transportiert werden.

Der in Figur 1 dargestellte Mehrfach-Kapillarsensor-Streifen 3 besteht aus elektrochemischen Kapillarsensoren, deren Elektroden zur Durchführung einer Messung mit einer Meß- und Auswerteelektronik 25 verbunden werden müssen. Zu diesem Zweck sind die Elektroden (wie später noch näher erläutert wird) mit im Innern der hohlen Trommel 6 aufgewickelten Drähten 26 verbunden, deren anderes Ende an entsprechende Sensorkontakte 27 am Boden der Kassette 8 angelötet sind. Die Sensorkontakte erstrecken sich auf die äußere Seite der Kassette 8 und stehen, wenn die Kassette 8 in das Auswertegerät 2 eingelegt ist, mit entsprechenden (in Figur 1 gestrichelt dargestellten) Gerätekontakten 28 in Verbindung, die wiederum über gerätefeste Leitungen 29 mit der Meß- und Auswerteelektronik 25 verbunden sind. Die Länge der Drähte 26 ist so gemessen, daß das Sensorband 4 vollständig abgewickelt werden kann.

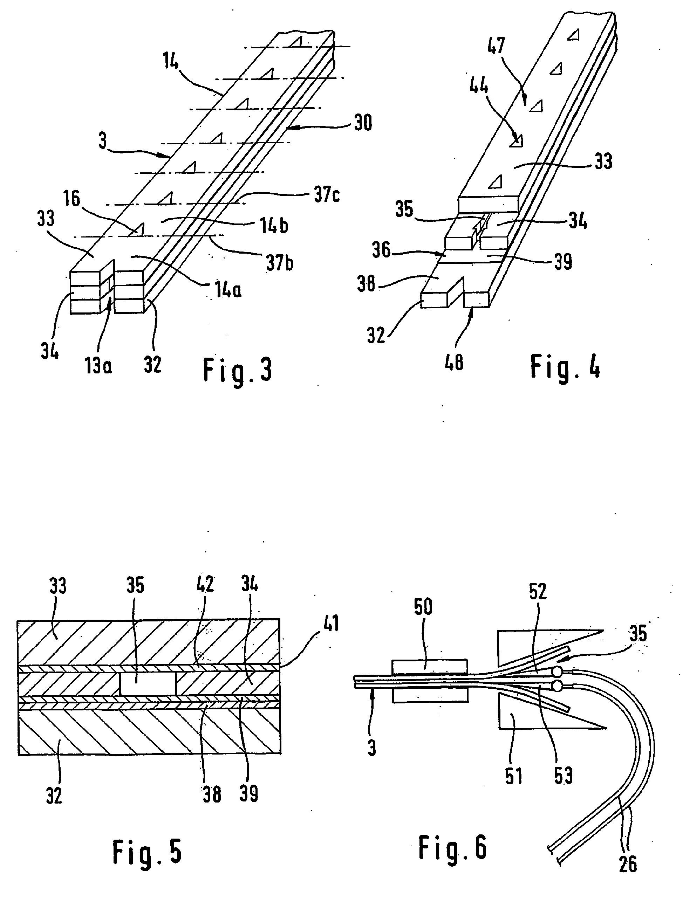

Einzelheiten eines bevorzugten Aufbaus der erfindungsgemäßen Mehrfach-Kapillarsensor-Streifen sind in den Figuren 3 bis 5 deutlicher zu erkennen. Die darin dargestellten Mehrfach-Kapillarsensor-Streifen 3 können als gerade langgestreckte Stange 30 ausgebildet sein. Bei Verwendung flexibler.Materialien von relativ geringer Dicke kann mit dem gleichen Aufbau jedoch auch ein flexibles Sensorband 4, wie in Figur 1 dargestellt, realisiert werden.

Strukturelle Elemente der dargestellten Konstruktion sind ein unteres Folienband 32, ein oberes Folienband 33 und zwei doppelseitig klebende Distanzstreifen 34, durch die die Folienbänder 32 und 33 im gewünschten Abstand voneinander derartig fixiert werden, daß ein durchgehender Kapillarkanal 35 entsteht. Der durchgehende Kapillarkanal 35 ist durch die Ausnehmungen 16 in die einzelnen Kapillarkanäle der Kapillarsensoren 14a, 14b usw. unterteilt. Nach oben und unten ist er von den Folienbändern 32,33 und seitwärts von den Distanzstreifen 34 begrenzt. Die Elemente 32 bis 34 bestehen aus in der Biosensor-Technik gebräuchlichen Kunststoffmaterialien, beispielsweise Polyester oder Polyamid.

Bei der dargestellten bevorzugten Ausführungsform elektrochemischer Biosensoren wird die Arbeitselektrode 36 der Sensoren 14 (deren Trennungslinien 37 in Figur 3 gestrichelt angedeutet sind) durch eine auf das untere Folienband 32 aufgedampfte Schicht 38 aus einem leitfähigen Material und eine darüberliegende Reagenzschicht 39 gebildet, die die notwendigen Reagenzien (zur Bestimmung von Glucose üblicherweise das Enzym Glucoseoxidase und einen geeigneten Mediator) enthält. Die Schicht 38 kann insbesondere aus einem Metall, wie beispielsweise Gold, Silber, Palladium oder Platin, oder aus Graphit bestehen. Die nur in Figur 5 erkennbare Gegenelektrode 41 kann beispielsweise von einer Ag/AgCl-Schicht 42 gebildet werden, die nur in Figur 5 erkennbar ist.

Eine derartige Konstruktion kann mit zur Herstellung üblicher Biosensoren gebräuchlichen Beschichtungs- und Verbindungstechniken kostengünstig als endloses Band hergestellt werden, wobei zunächst die Folien 32 und 33 mit den Schichten 38, 39 bzw. 42 beschichtet und dann mittels der doppelseitig klebenden Distanzstreifen 34 verbunden werden.

Nach Fertigstellung der Sandwich-Konstruktion werden die Ausnehmungen 16 eingestanzt, die in dem durchgehenden Kapillarkanal 35 jeweils ein Strömungshinderniss 44 bilden. Durch diese Strömungshindernisse 44 wird die Strömung einer durch die Einlaßöffnung 13a in den vordersten Kapillarsensor 14a eindringenden Probenflüssigkeit gestoppt und zuverlässig verhindert, daß die Probenflüssigkeit in den nächstfolgenden Kapillarsensor 14b des Mehrfach-Kapillarsensor-Streifens 3 eindringt. Dabei entweicht die verdrängte Luft durch die Ausnehmung 16, die gleichzeitig die Entlüftungsöffnung 17 des davorliegenden Kapillarsensors 14a und (nach dem Abschneiden des vordersten Kapillarsensors längs der Trennungslinie 37b) die Eintrittsöffnung 13 des nächstfolgenden Kapillarsensors 14 bildet. Die Herstellung wird bei der dargestellten Ausführungsform auch dadurch vereinfacht, daß es im Produktionsprozeß auf die Relativposition der miteinander zu dem Sandwich verbundenen Bänder in Längsrichtung nicht ankommt.

Bei der dargestellten bevorzugten Ausführungsform werden die Wandteile 47,48, die die Kapillarkanäle 15 der Kapillarsensoren 14 begrenzen, von flachen nicht profilierten Folien gebildet und erstrecken sich über die gesamte Länge des Mehrfach-Kapillarsensor-Streifens 3. Eine solche Konstruktion hat die erwähnten produktionstechnischen Vorteile. Außerdem kann eine hohe Flexibilität erreicht werden, so daß die Kapillarsensor-Streifen mit engem Krümmungsradius gerollt und dadurch platzsparend in das Analysesystem integriert werden können. Die Mehrfach-Kapillarsensor-Streifen können im Prinzip jedoch auch aus profilierten Teilen, beispielsweise tiefgezogenen profilierten Folien hergestellt sein, wobei die Profilierung jedoch vorzugsweise nicht so stark sein sollte, daß die gewünschte Flexibilität nicht mehr gegeben ist.

In jedem Fall bestehen die Wandteile, die den Kapillarkanal umschließen, bevorzugt aus Kunststoff-Folienbändern, die von Rollen abgezogen und in einem kontinuierlichen Verfahren derartig miteinander verbunden werden können, daß zwischen ihnen der Kapillarspalt verbleibt. Die erforderliche Trennung der Kapillarspalten aufeinanderfolgender Kapillarsensoren voneinander kann - statt durch die Ausnehmungen 16 - auch dadurch erreicht werden, daß im Rahmen eines solchen Produktionsprozesses Distanzstreifen verwendet werden, die nicht nur entlang der Längskanten verlaufen, sondern Querstege aufweisen, durch die die Kapillarkanäle der einzelnen Sensoren voneinander getrennt werden. In diesem Fall erfolgt die Entlüftung zweckmäßigerweise über ein Entlüftungsloch, das in dem oberen Folienband jeweils am Ende des Kapillarkanals jedes Sensors vorgesehen ist.

Bei der dargestellten Ausführungsform erstrecken sich die von der Goldschicht 38 bzw. der Ag/AgCl-Schicht 42 gebildeten Leiterbahnen ohne galvanische Trennung jeweils über sämtliche Kapillarsensoren 14. des Mehrfach-Kapillarsensor-Streifens 3 und sind über einen gemeinsamen Sensorkontakt 27 mit einem entsprechenden Gerätekontakt 28 und dadurch mit der Meß- und Auswerteelektronik des Auswertegerätes verbunden (Figur 1). Figur 6 zeigt eine hierfür geeignete Konstruktion für die elektrische Kontaktierung zwischen dem Mehrfach-Kapillarsensor-Streifen 3 und den Verbindungsdrähten 26, die zu der Meß- und Auswerteelektronik führen. Zur Montage wird der Mehrfach-Kapillarsensor-Streifen 3 in eine Führung 50 geschoben, die ein Bestandteil der in Figur 1 dargestellten Trommel 6 sein kann. Beim Eindringen des Streifens 3 in eine Steckvorrichtung 51 dringen zwei keilförmig gegeneinander angeordnete elektrisch voneinander isolierte Kontaktschneiden in den Kapillarkanal 35 des Streifens 3 ein, spalten diesen auf und kontaktieren dabei dessen leitfähige Innenseiten. Die beiden Kontaktschneiden 52, 53 sind mit den Drähten 26 verbunden.

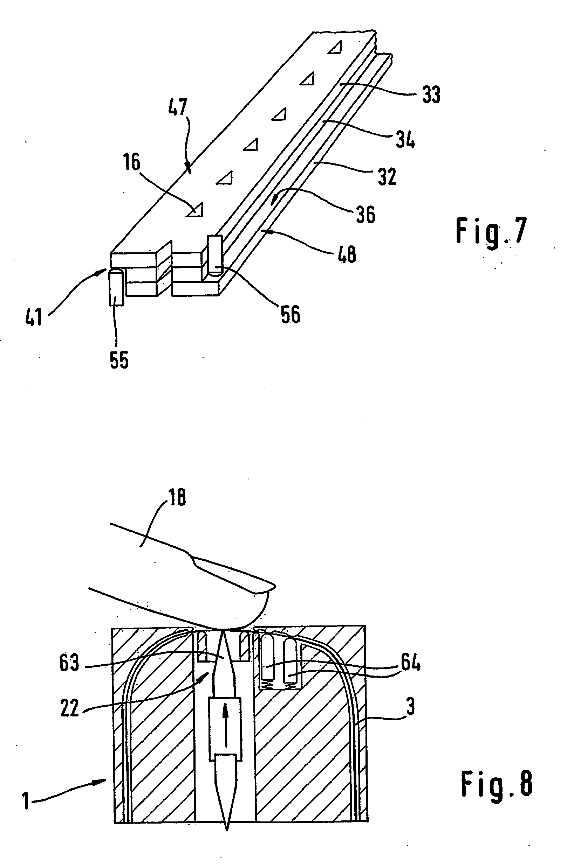

Der in Figur 7 dargestellte Mehrfach-Kapillarsensor-Streifen 3 unterscheidet sich von der Ausführungsform gemäß der Figuren 3 bis 5 dadurch, daß der elektrische Kontakt zwischen den Elektroden 36, 41 mittels gerätefest gelagerten Schleifkontakten 55, 56 hergestellt wird, die in unmittelbarem Kontakt zu den entsprechenden Elektrodenbahnen stehen.

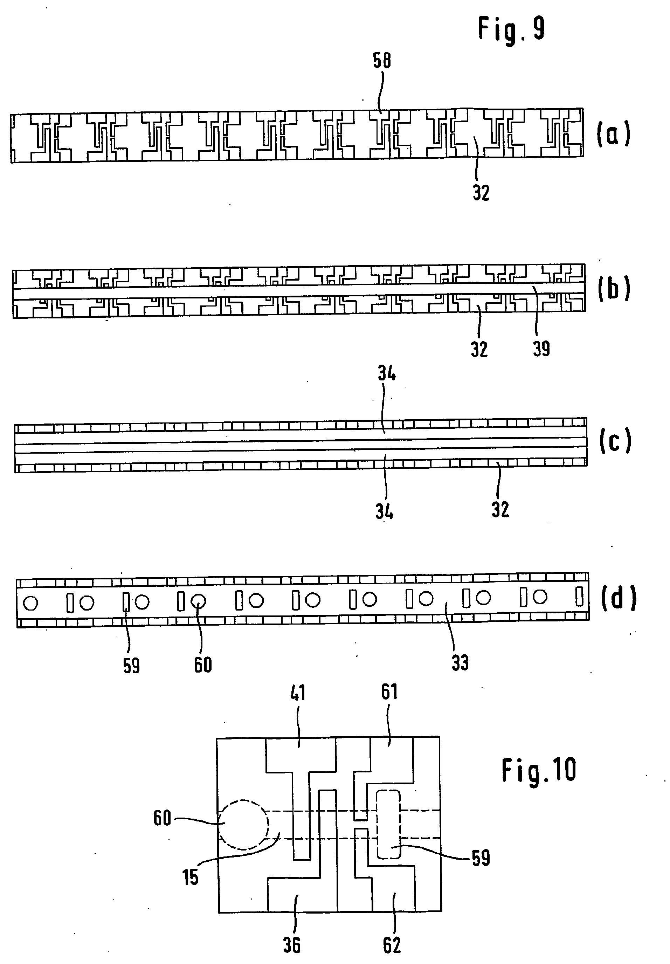

Bei der in den Figuren 9 und 10 dargestellten alternativen Ausführungsform werden statt vollflächiger Elektroden in gegenüberliegender Anordnung strukturierte Elektroden in einer Ebene verwendet. Auch ein solcher Mehrfach-Kapillarsensor-Streifen kann als Sandwichkonstruktion mit drei strukturellen Schichten realisiert werden, wobei die Wandteile, die den Kapillarkanal nach oben und unten begrenzen, von Folienbändern gebildet werden.

Das Layout der Schichten ist in Figur 9 dargestellt. Die Teilfiguren a, b und c zeigen eine Aufsicht auf das untere Folienband 32 in drei Herstellungsstufen. In der Teilfigur a erkennt man eine Leiterstruktur 58 für die Elektroden, in der Teilfigur b eine darüber verlaufende Reagenzschicht 39. Teilfigur c zeigt das untere Folienband 32 nach Anbringung von zwei Distanzstreifen 34. Teilfigur d schließlich zeigt ein oberes Folienband 33 mit eingestanzten Löchern 59 und 60. Die Löcher 60 dienen der Probenzuführung und die Löcher 59 der Entlüftung. Das Entlüftungsloch 59 bildet gleichzeitig ein Strömungshindernis, durch das die Strömung längs des Kapillarkanals gestoppt und dadurch verhindert wird, daß die Probenflüssigkeit in den benachbarten Kapillarsensor eindringen kann. Hierzu genügt eine Ausnehmung, die mindestens eines der Wandteile bis zu dem Kapillarkanal durchdringt und sich über dessen gesamte Breite erstreckt. Alternativ kann sich aber sowohl die als Entlüftungsausnehmung als auch die zur Probenzuführung dienende Ausnehmung durch beide Wandteile, also durch den gesamten Sensor, erstrecken.

Wie aus Figur 10 deutlicher zu ersehen ist, sind bei dieser Ausführungsform neben der Arbeitselektrode 36 und der Gegenelektrode 41 zusätzliche Elektroden 61 und 62 vorgesehen, die beispielsweise zur Kontrolle der Befüllung dienen können. Figur 10 zeigt auch (in gestrichelter Darstellung) die bevorzugte Position der Löcher 59 und 60. Wenn ein Blutstropfen mit dem als Einfüllöffnung dienenden Loch 59 kontaktiert wird, wird er in den Kapillarkanal 15 gesaugt, wobei er zunächst die Elektroden 36 und 41 kontaktiert. Beim weiteren Eindringen kontaktiert die Probe die Kontrollelektroden 61 und 62. Dadurch sinkt der elektrische Widerstand zwischen diesen Elektroden. Diese Widerstandsänderung kann auf bekannte Weise als Signal dafür gemessen werden, daß der Kapillarkanal 15 bis zu dem Bereich der Kontrollelektroden 61,62 gefüllt und somit ein ausreichender Kontakt zu der Arbeitselektrode 36 und der Gegenelektrode 41 gewährleistet ist. Um diese Funktion sicherzustellen, müssen die Kontrollelektroden 61,62 zwischen den Elektroden 36,41 und der Entlüftungsöffnung 59 positioniert sein. Die dargestellten relativ komplizierte Elektrodenstrukturen können rationell in einem kontinuierlichen "Laserablation-Prozeß" erzeugt werden.

Bei der in Figur 8 schematisch,dargestellten Ausführungsform eines entsprechenden Analysesystems 1 wird der Mehrfach-Kapillarsensor-Streifen 3 schrittweise kontinuierlich an der Meßposition 22 vorbeigeführt. An der Meßposition 22 ist eine mechanisch bewegliche Lanzette 63 vorgesehen, die einen Stich in den Finger 18 ausführt. Der dabei gewonnene Blutstropfen dringt durch ein Loch 65 in den in der Meßposition 22 befindlichen Kapillarsensor ein. Das dabei gewonnene elektrische Signal wird über Elektrodenkontakte 64 abgegriffen und der Meß- und Auswerteschaltung zugeführt. Der hier verwendete Mehrfach-Kapillarsensor-Streifen 3 kann gemäß den Figuren 9 und 10 gestaltet sein, jedoch kann auch eine Gestaltung mit vollflächigen Reagenzschichten, wie bei den Figuren 3 bis 5 dargestellt, verwendet werden. Jedenfalls muß sich die als Einlaßöffnung dienende Ausnehmung durch alle Schichten des Sensors erstrecken, damit die Lanzette 63 durch diese Ausnehmung hindurch in den Finger einstechen kann.

Aus dem US-Patent 5,047,044 ist eine Sensorkonstruktion bekannt, bei der Lanzetten jeweils integraler Bestandteil der Sensorkonstruktion sind. Dies erfordert jedoch eine Gestaltung des Sensors aus kompliziert geformten, in der Praxis nur im Spritzgußverfahren herstellbaren Formteilen mit vielen Einzelelementen. Demgegenüber ist die in Figur 10 dargestellte Gestaltung sehr viel einfacher und kostengünstiger.

## Patentansprüche

1. Kapillarsensor-Analysesystem zur Analyse einer Probenflüssigkeit auf einen darin enthaltenen Analyten, insbesondere zur Analyse einer Körperflüssigkeit von Menschen oder Tieren, mit Kapillarsensoren (14),
- die einen von mindestens zwei Wandteilen (42,48) umschlossenen Kapillarkanal (15) mit einer Einlaßöffnung (13) für die Probenflüssigkeit (19) und einer Entlüftungsöffnung (17) aufweisen,
- in deren Kapillarkanal (15) Reagenzien enthalten sind, wobei die Reaktion der Probenflüssigkeit (19) mit den Reagenzien zu einer meßbaren Änderung einer für die Analyse charakteristischen Meßgröße führt,
und einem Auswertegerät (2), das
- eine Kapillarsensorhalterung (23) aufweist, um einen Kapillarsensor (14) in einer Meßposition (22) zur Durchführung einer Analyse so zu positionieren, daß seine Einlaßöffnung (13) zugänglich ist, um eine zu untersuchende Probenflüssigkeit (19) mit der Einlaßöffnung (13) in Kontakt zu bringen, wobei die Probenflüssigkeit (19) aufgrund von Kapillarkräften in den Kapillarkanal (15) eindringt und diesen füllt, und
- eine Meß- und Auswerteelektronik (25) zur Messung der Meßgröße und Ermittlung der gewünschten Analyseinformation auf Basis des aus der Messung resultierenden Meßwertes einschließt,
wobei
die Kapillarsensoren (14) als Mehrfach-Kapillarsensor-Streifen (3) mit einer Mehrzahl von hintereinander angeordneten Kapillarsensoren (14) ausgebildet sind,
ein Mehrfach-Kapillarsensor-Streifen (3) in der Kapillarsensor-Halterung (23) des Auswertegerätes (2) so geführt und gehalten wird, daß sich jeweils ein Kapillarsensor (14a) des Streifens (3) in der Meßposition (22) befindet und seine Einlaßöffnung (13a) zur Kontaktierung mit Probenflüssigkeit (19) zugänglich ist, und
der Mehrfach-Kapillarsensor-Streifen (3) in dem Auswertegerät (2) so bewegbar ist, daß nacheinander aufeinanderfolgende Kapillarsensoren (14b,14c ...) des Mehrfach-Kapillarsensor-Streifens (3) in die Meßposition (22) transportiert werden,
**dadurch gekennzeichnet dass**
die Kapillarkanäle der Mehrfach-Kapillarsensor-Streifen ein Strömungshindernis (44) einschließen, das durch eine mindestens eines der Wandteile (47,48) durchdringende Ausnehmung (16,59), die sich über die Breite des Kapillarkanals (15) erstreckt, gebildet wird und durch das die Strömung einer durch seine Einlaßöffnung (15a) eindringenden Probenflüssigkeit gestoppt und **dadurch** verhindert wird, daß die Probenflüssigkeit in den nächstfolgenden Kapillarsensor (14) des Mehrfach-Kapillarsensor-Streifens (3) eindringt.

2. Kapillarsensor-Analysesystem nach Anspruch 1, wobei das Auswertegerät (2) eine Schneideeinrichtung (20) aufweist, durch die jeweils nach Durchführung einer Messung der bei der Messung benutzte Kapillarsensor (15) von dem Mehrfach-Kapillarsensor-Streifen (3) abgeschnitten wird.

3. Kapillarsensor-Analysesystem nach einem der vorhergehenden Ansprüche, wobei die Kapillarsensoren (14) des Mehrfach-Kapillarsensor-Streifens als elektrochemische Kapillarsensoren ausgebildet sind, die jeweils eine Arbeitselektrode (36), eine Gegenelektrode (41) und mit den Elektroden über Leiterbahnen (38,42) verbundene Sensorkontakte (27) aufweisen, die während der Messung mit entsprechenden Gerätekontakten (28) des Auswertegerätes (2) in Kontakt stehen, um eine elektrische Verbindung zu der Meß- und Auswerteelektronik (25) herzustellen.

4. Kapillarsensor-Analysesystem nach Anspruch 3, wobei sich die Leiterbahnen (38,42) mindestens einer der Elektroden über sämtliche Kapillarsensoren des Mehrfach-Kapillarsensor-Streifens (3) ohne galvanische Trennung erstrecken und über einen gemeinsamen Sensorkontakt (27) mit einem entsprechenden Gerätekontakt (28) und **dadurch** mit der Meß- und Auswerteelektronik (25) des Auswertegerätes (2) verbunden sind.

5. Mehrfäch-Kapillarsänsor-Streifen für ein Analysesystem nach einem der Ansprüche 1 bis 4, mit einer Mehrzahl von miteinander verbundenen, hintereinander angeordneten Kapillarsensoren (14),
wobei
die Kapillarsensoren (14) jeweils einen von mindestens zwei Wandteilen (47,48) umschlossenen Kapillarkanal (15) mit einer Einlaßöffnung (13) für die Probenflüssigkeit (19) und einer Entlüftungsöffnung (17) aufweisen,
die Kapillarkanäle der Kapillarsensoren ein Strömungshindernis (44) einschließen, das durch eine mindestens eines der Wandteile (47,48) durchdringende Ausnehmung (16,59), die sich über die Breite des Kapillarkanals (15) erstreckt, gebildet wird und durch das die Strömung einer durch seine Einlaßöffnung (15a) eindringenden Probenflüssigkeit gestoppt und **dadurch** verhindert wird, daß die Probenflüssigkeit in den nächstfolgenden Kapillarsensor (14) des Mehrfach-Kapillarsensor-Streifens (3) eindringt, und
in dem Kapillarkanal (15) der Kapillarsensoren (14) jeweils Reagenzien enthalten sind, wobei die Reaktion der Probenflüssigkeit (19) mit mindestens einem der Reagenzien zu einer meßbaren Änderung einer für die Analyse charakteristischen Meßgröße führt.

6. Mehrfach-Kapillarsensor-Streifen nach Anspruch 5, bei welchem die Wandteile (47,48) aus Folienbändern gebildet sind.

7. Mehrfach-Kapillarsensor-Streifen nach Anspruch 5 oder 6, welcher als flexibles, in dem Auswertegerät (2) in gekrümmtem Zustand aufgewickeltes Band (4) ausgebildet ist.

8. Mehrfach-Kapillarsensor-Streifen nach einem der Ansprüche 5 oder 6, welcher zwei durchgehende Wandteile (47,48) aufweist, die sich über die gesamte Länge der hintereinander angeordneten Kapillarsensoren erstrecken und die Kapillarkanäle (15) aller Kapillarsensoren (14) des Mehrfach-Kapillarsensor-Streifens umschließen.

9. Mehrfach-Kapillarsensor-Streifen nach einem der Ansprüche 5 bis 8, wobei die Kapillarkanäle (15) der Kapillarsensoren (14) in dem Mehrfach-Kapillarsensor-Streifen (3) fluchtend aneinander anschließen, so daß ihre Einlaßöffnungen (13) und Enttüftungsöffnungen (17) einander gegenüberliegen.

10. Mehrfach-Kapillarsensor-Streifen nach einem der Ansprüche 5 bis 9, welcher als elektrochemischer Kapillarsensor ausgebildet ist und eine Arbeitselektrode (36), eine Gegenelektrode (41) und mit den Elektroden (36, 41) verbundene Sensorkontakte (27) aufweist, um eine elektrische Verbindung mit einer zur Messung der elektrischen Meßgröße geeigneten Meßelektronik (25) herzustellen.

11. Mehrfach-Kapillarsensor-Streifen nach Anspruch 10, wobei eine elektrische Leiterbahn (38) der Arbeitselektroden (36) der Kapillarsensoren (14) an der Oberfläche des einen Wandteils (48) und eine elektrische Leiterbahn (42) der Gegenelektroden (41) der Kapillarsensoren (14) an der Oberfläche des anderen Wandteils (47) verläuft.

## Claims

1. A capillary sensor analysis system for analyzing a sample liquid with respect to an analyte contained therein, in particular for analyzing a body fluid of humans.or animals, comprising
capillary sensors (14),
including a capillary channel (15), which is delimited by at least two wall parts (42, 48) and has an inlet opening (13) for the sample liquid (19) and a vent opening (17),
the capillary channel (15) containing reagents, the reaction of the sample liquid (19) with the reagents resulting in a measurable change of a measurement variable which is characteristic for the analysis,
and an evaluation instrument (2), which includes
a capillary sensor holder (23) suitable for positioning a capillary sensor (14) for performing an analysis in a measurement position (22) in such a manner that its inlet opening (13) is accessible for bringing a sample liquid (19) to be assayed into contact with the inlet opening (13), whereby the sample liquid (19) enters and fills, driven by capillary forces, the capillary channel (15), and
a measurement and evaluation electronics (25) for measuring the measurement variable and determining the desired analysis information on the basis of the measured value resulting from the measurement,
wherein
the capillary sensors (14) are shaped as multiple capillary sensor strips (3) comprising a plurality of capillary sensors (14) arranged one after the other,
a multiple capillary sensor strip (3) is guided and held in the capillary sensor holder (23) of the evaluation instrument (2) in such a manner that one capillary sensor (14a) of the strip (3) at a time is located in the measurement position (22) and its inlet opening (13a) is accessible for contact with sample liquid (19), and
the multiple capillary sensor strip (3) is movable in the evaluation instrument (2) in such a manner that consecutive capillary sensors (14b, 14c,...) of the multiple capillary sensor strip (3) are transported one after the other into the measurement position (22),
**characterized in that**
the capillary channels (15) of the multiple capillary sensor strip (3) include a flow obstruction (44) formed by a recess (16,59) which extends through at least one of the wall parts (47, 48) and over the width of the capillary channel (15), by which flow obstruction the flow of a sample liquid entering via its inlet opening (15a) is stopped and the sample liquid is thus prevented from advancing into the next following capillary sensor (14) of the multiple capillary sensor strip (3).

2. Capillary sensor analysis system according to claim 1, wherein the evaluation instrument (2) has a cutting device (20), by which, after performing a measurement, the capillary sensor (15) used for that measurement is cut off from the multiple capillary sensor strip (3).

3. Capillary sensor analysis system according to any one of the preceding claims, wherein the capillary sensors (14) of the multiple capillary sensor strip are electrochemical capillary sensors, each of which has a working electrode (36), a counter electrode (41), and sensor contacts (27) connected to the electrodes via conducting leads (38, 42), the sensor contacts being during the measurement in contact with corresponding mating contacts (28) of the evaluation instrument (2) for providing an electrical connection to the measurement and evaluation electronics (25).

4. Capillary sensor analysis system according to claim 3, wherein the conducting leads (38, 42) of at least one of the electrodes extend, without electrical separation, over all capillary sensors of the multiple capillary sensor strip (3) for connection via a common sensor contact (27) to a corresponding instrument contact (28) and thus to the measurement and evaluation electronics (25) of the evaluation instrument (2).

5. Multiple capillary sensor strip for an analysis system according to any one of claims 1 to 4, comprising a plurality of capillary sensors (14) which are connected to one another and arranged one after the other,
wherein
the capillary sensors (14) each include a capillary channel (15) which is delimited by at least two wall parts (47, 48) and has an inlet opening (13) for the sample liquid (19) and a vent opening (17),
the capillary channels (15) of the multiple capillary sensor strip (3) include a flow obstruction (44) formed by a recess (16,59) which extends through at least one of the wall parts (47, 48) and over the width of the capillary channel (15), by which flow obstruction the flow of a sample liquid entering via its inlet opening (15a) is stopped and the sample liquid is thus prevented from advancing into the next following capillary sensor (14) of the multiple capillary sensor strip (3), and
reagents are contained in the capillary channel (15) of each of the capillary sensors (14), the reaction of the sample liquid (19) with at least one of the reagents resulting in a measurable change of a measurement variable which is characteristic for the analysis.

6. Multiple capillary sensor strip according to claim 5, wherein the wall parts (47, 48) are formed from film bands.

7. Multiple capillary sensor strip according to any one of claims 5 or 6, which is implemented as a flexible band (4), wound up in the evaluation instrument (2) in the curved state.

8. Multiple capillary sensor strip according to any one of claims 5 or 6, which has two continuous wall parts (47, 48) extending over the total length of the capillary sensors arranged one after the other and delimiting the capillary channels (15) of all capillary sensors (14) of the multiple capillary sensor strip.

9. Multiple capillary sensor strip according to any one of claims 5 to 8, wherein the capillary channels (15) of the capillary sensors (14) in the multiple capillary sensor strip (13) adjoin one another in alignment in such a manner that their inlet openings (13) and vent openings (17) face one another.

10. Multiple capillary sensor strip according to any one of claims 5 to 9, which is an electrochemical capillary sensor and has a working electrode (36), a counter electrode (41), and sensor contacts (27) connected to the electrodes (36, 41) for providing an electrical connection to a measurement electronics (25) suitable for measuring the electrical measurement variable.

11. Multiple capillary sensor strip according to claim 10, wherein an electrical conducting lead (38) of the working electrodes (36) of the capillary sensors (14) runs on the surface of one wall part (48) and an electrical conducting lead (42) of the counter electrodes (41) of the capillary sensors (14) runs on the surface of the other wall part (47).

## Revendications

1. Système d'analyse à capteurs capillaires pour l'analyse d'un liquide d'échantillon sur un analyte contenu dedans, en particulier pour l'analyse d'un liquide du corps d'êtres humains ou d'animaux, comprenant
des capteurs capillaires (14)
- qui présentent un canal capillaire (15) entouré d'au moins deux parties de paroi (42,48), avec un orifice d'entrée (13) pour le liquide d'échantillon (19) et un orifice d'aération (17)
- dans le canal capillaire(15) desquels sont contenus des réactifs, la réaction du liquide d'échantillon (19) avec les réactifs entraînant un changement mesurable d'une grandeur de mesure caractéristique pour l'analyse,
et un appareil d'analyse (2)
- qui présente un support de capteur capillaire (23), afin de positionner un capteur capillaire (14) pour effectuer une analyse dans une position de mesure (22), de sorte que son orifice d'entrée (13) est accessible, afin de mettre en contact le liquide d'échantillon (19) à analyser avec l'orifice d'entrée (13), le liquide d'échantillon (19) pénétrant, du fait des forces capillaires, dans le canal capillaire (15) et remplissant ce dernier, et
- qui comprend un organe électronique d'analyse et de mesure (25) pour la mesure de la grandeur de mesure et la détermination de l'information d'analyse requise sur la base de la valeur de mesure résultant de la mesure,
dans lequel les capteurs capillaires (14) sont conformés en une bande à capteurs capillaires multiples (3) avec une pluralité de capteurs capillaires (14) disposés en série,
une bande à capteurs capillaires multiples (3) est guidée et maintenue dans le support à capteurs capillaires (23) de l'appareil d'analyse (2) de sorte qu'un capteur capillaire (14a) de la bande (3) se trouve respectivement dans la position de mesure (22) et que son orifice d'entrée (13a) est accessible pour la mise en contact avec le liquide d'échantillon (19), et
la bande à capteurs capillaires multiples (3) est être mue dans l'appareil d'analyse (2) de sorte que des capteurs capillaires (14b,14c...) de la bande à capteurs capillaires multiples (3) qui se succèdent les uns les autres sont transportés dans la position de mesure (22),
**caractérisé en ce que**
les canaux capillaires de la bande à capteurs capillaires multiples comprennent un obstacle entravant le flux (44) qui est formé par un évidement (16,59) pénétrant à travers au moins l'une des parties de parois (47,48), lequel s'étend sur la largeur du canal capillaire (15) et par lequel le flux d'un liquide d'échantillon pénétrant par l'orifice d'entrée (15a) est stoppé, et que l'on empêche de ce fait que le liquide d'échantillon ne pénètre dans le capteur capillaire subséquent (14) de la bande à capteurs capillaires multiples (3).

2. Système d'analyse à capteurs capillaires selon la revendication 1, dans lequel l'appareil d'analyse (2) présente un dispositif de coupe (20) par lequel après l'exécution de chaque mesure respective, le capteur capillaire (15) utilisé lors de la mesure est découpé de la bande à capteurs capillaires multiples (3).

3. Système d'analyse à capteurs capillaires selon l'une quelconque des revendications précédentes, dans lequel les capteurs capillaires (14) de la bande à capteurs capillaires multiples sont conformés en tant que capteurs capillaires électrochimiques qui présentent respectivement une électrode de travail (36), une contre-électrode (41) et des contacts de capteurs (27) reliés aux électrodes via des pistes conductrices (38,42), lesquels contacts sont pendant la mesure en contact avec les contacts d'appareils correspondants (28) de l'appareil d'analyse (2), afin d'établir une liaison électrique avec l'organe électronique de mesure et d'analyse (25).

4. Système d'analyse à capteurs capillaires selon la revendication 3, dans lequel les pistes conductrices (38,42) d'au moins une électrode s'étendent sur tous les capteurs capillaires de la bande à capteurs capillaires multiples (3) sans séparation galvanique et sont reliées, via un contact de capteur (27) commun, à un contact d'appareil (28) correspondant et de ce fait à l'organe électronique d'analyse et de mesure (25) de l'appareil d'analyse (2).

5. Bande à capteurs capillaires multiples pour un système d'analyse selon l'une quelconque des revendications 1 à 4, comprenant une pluralité de capteurs capillaires (14) disposés en série et reliés entre eux
dans lequel
les capteurs capillaires (14) présentent respectivement un canal capillaire (15) entouré d'au moins deux parties de parois (47,48), avec un orifice d'entrée (13) pour le liquide d'échantillon (19) et un orifice d'aération (17),
les canaux capillaires des capteurs capillaires (14) comprennent un obstacle entravant le flux (44) qui est formé par un évidement (16,59) pénétrant à travers au moins l'une des parties de parois (47,48), lequel s'étend sur la largeur du canal capillaire (15) et par lequel le flux d'un liquide d'échantillon pénétrant par son orifice d'entrée (15a) est stoppé, et que l'on empêche de ce fait que le liquide d'échantillon ne pénètre dans le capteur capillaire subséquent (14) de la bande à capteurs capillaires multiples (3), et
le canal capillaire (15) des capteurs capillaires (14) contient respectivement des réactifs, la réaction du liquide d'échantillon (19) avec au moins l'un des réactifs entraînant un changement mesurable d'une grandeur de mesure caractéristique pour l'analyse.

6. Bande à capteurs capillaires multiples selon la revendication 5, dans laquelle les parties de parois (47,48) sont constituées par des bandes de films.

7. Bande à capteurs capillaires multiples selon la revendication 5 ou 6, laquelle est conformée comme une bande flexible (4) enroulée à l'état cintré dans l'appareil d'analyse (2).

8. Bande à capteurs capillaires multiples selon l'une quelconque des revendications 5 ou 6, laquelle présente deux parties de parois continues (47,48) qui s'étendent sur toute la longueur des capteurs capillaires disposés en série, et englobent les canaux capillaires (15) de tous les capteurs capillaires (14) de la bande à capteurs capillaires multiples.

9. Bande à capteurs capillaires multiples selon l'une quelconque des revendications 5 à 8, dans laquelle les canaux capillaires (15) des capteurs capillaires (14) sont alignés de bout en bout dans la bande à capteurs capillaires multiples (3), de sorte que leurs orifices d'entrée (13) et leurs orifices d'aération (17) se font face les uns les autres.

10. Bande à capteurs capillaires multiples selon l'une quelconque des revendications 5 à 9, laquelle est conformée en tant que capteur capillaire électrochimique et présente une électrode de travail (36) une contre-électrode (41) et des contacts de capteurs (27) reliés avec les électrodes (36,41), afin d'établir une liaison électrique avec un organe électronique de mesure (25) convenant à la mesure de la grandeur de mesure électrique.

11. Bande à capteurs capillaires multiples selon la revendication 10, dans laquelle une piste conductrice électrique (38) des électrodes de travail (36) des capteurs capillaires (14) parcourt la surface de l'une des parties de paroi (48) et une piste conductrice électrique (42) des contre-électrodes (41) des capteurs capillaires (14) parcourt la surface de l'autre partie de paroi (47).
